Europäisches Patentamt

European Patent Office

Office européen des brevets

(11) Publication number: **0 034 542**
**B1**

(12) **EUROPEAN PATENT SPECIFICATION**

(45) Date of publication of patent specification: **28.08.85**

(51) Int. Cl.⁴: **C 07 D 285/10, A 61 K 31/41**

(21) Application number: **81400251.5**

(22) Date of filing: **18.02.81**

(54) **S-(-)-1-(tert-butylamino)-3-((4-morpholino-1,2,5-thiadiazol-3-yl)-oxy)-2-propanol pamoate for topical application in the treatment of elevated intraocular pressure.**

| | |
|---|---|
| (30) Priority: **19.02.80 US 122338** | (73) Proprietor: **MERCK & CO. INC.**<br>**126, East Lincoln Avenue P.O. Box 2000**<br>**Rahway New Jersey 07065 (US)** |
| (43) Date of publication of application:<br>**26.08.81 Bulletin 81/34** | (72) Inventor: **Henley, Martin W.**<br>**RD 2 Box 223 Lower Mountain Road**<br>**New Hope Pennsylvania 18938 (US)** |
| (45) Publication of the grant of the patent:<br>**28.08.85 Bulletin 85/35** | Inventor: **Harwood, Richard J.**<br>**3219 Adams Court North**<br>**Bensalem Pennsylvania 19020 (US)** |
| (84) Designated Contracting States:<br>**AT BE CH DE FR GB IT LI LU NL SE** | (74) Representative: **Corre, Jacques Denis Paul et al**<br>**Cabinet Regimbeau 26, Avenue Kléber**<br>**F-75116 Paris (FR)** |
| (56) References cited:<br>**CH-A- 597 209**<br>**DE-A-2 428 030**<br>**FR-A-2 277 581**<br>**GB-A-1 253 709**<br>**GB-A-1 264 558**<br>**GB-A-1 385 648**<br>**GB-A-1 429 420** | |

Courier Press, Leamington Spa, England.

**0 034 542**

**Description**

Disclosure of the invention

This invention relates to S - (−) - 1 - (*tert* - butylamino) - 3 - [(4 - morpholino - 1,2,5 - thiadiazol - 3 - yl)oxy] - 2 - propanol pamoate or its racemic modification for the treatment of elevated intraocular pressure.

In particular, this invention relates to compositions of S - (−) - 1 - (*tert* - butylamino) - 3 - [(4 - morpholino - 1,2,5 - thiadiazol - 3 - yl)oxy] - 2 - propanol pamoate and its use for lowering intraocular pressure, especially in the treatment of ocular hypertension and glaucoma.

Glaucoma is a degenerative disease of the eye wherein the pressure in the eye (i.e., intraocular pressure) is too high for the normal function of the eye and, as a result, damage occurs to the optic nerve head and results in irreversible loss of visual function. If untreated, glaucoma will eventually lead to blindness. Ocular hypertension, i.e., the condition of elevated intraocular pressure without optic nerve head damage or characteristic glaucomatous visual field loss, is now believed by the majority of ophthalmologists to merely represent the earliest phase in the onset of glaucoma.

A patient who is in the very early stages of the onset of glaucoma and is experiencing no overt discomfort symptoms will even, with high motivation, tend to neglect the regular administration of medication. At this stage, no pathogen has as yet developed and only elevated intraocular pressure is present. Thus, ocular hypertension may be present for an extended period prior to reaching the stage of pathological damage.

When used to lower intraocular pressure, according to the present invention, S - (−) - 1 - (*tert* - butylamino) - 3 - [(4 - morpholino - 1,2,5 - thiadiazol - 3 - yl)oxy] - 2 - propanol and its racemic modifications are administered topically in insert form as the acid addition salt derived from pamoic acid.

In the ophthalmic formulation, from 0.01% to 50% by weight of S - (−) - 1 - (*tert* - butylamino) - 3 - [(4 - morpholino - 1,2,5 - thiadiazol - 3 - yl)oxy] - 2 - propanol pamoate is employed preferably from 1.0% to 50% of S - (−) - 1 - (*tert* - butylamino) - 3 - [(4 - morpholino - 1,2,5 - thiadiazol - 3 - yl)oxy] - 2 - propanol pamoate is most suitably employed.

In this specification, the term S - (−) - 1 - (*tert* - butylamino) - 3 - [(4 - morpholino - 1,2,5 - thiadiazol - 3 - yl) - oxy] - 2 - propanol pamoate refers to the 2:1 salt. That is, two moles of the S - (−) - 1 - (*tert* - butylamino) - 3 - [(4 - morpholino - 1,2,5 - thiadiazol - 3 - yl) - oxy] - 2 - propanol pamoate are present for each mole of pamoic acid.

Timolol is described in GB—A—1253709 as β adrenergic blocking agent and the pamoate is mentioned as suitable pharmacologically acceptable salts of such compound.

The dosage forms of this invention are administered in the form of ophthalmic solid inserts adapted for topical administration to the eye.

Formulations of these compounds may contain from 0,01 to 50% by weight. Higher dosages as, for example, about 10%, or lower dosages can be employed provided the dose is effective in lowering intraocular pressure. As a unit dosage from between 0,001 to 1.0 mg. is generally applied to the human eye, generally on a daily basis.

The pharmaceutical preparation of the invention are in the form of a solid insert. For example, one may use a solid, water soluble polymer as the carrier for the medicament. Inserts that are known in the art that are suitable for use with this combination include those set forth and described in U.S.P. 3,993,071; U.S.P. 3,986,510; U.S.P. 3,868,445; and U.S.P. 3,867,510 employing the formulation and fabrication techniques described therein. The polymer used to form the insert may be any water soluble non-toxic polymer, for example, cellulose derivatives such as methyl cellulose, alkali metal carboxymethyl cellulose, hydroxyethyl cellulose, hydroxypropyl cellulose, hydroxypropylmethyl cellulose; acrylates such as polyacrylic acid salts, ethyl acrylates and polyacrylamides; natural products such as gelatin, alginates, pectins, tragacanth, karaya, chondrus, agar, acacia, the starch derivatives such as starch acetate, hydroxyethyl starch ethers, hydroxypropyl starch, as well as other synthetic derivatives such as polyvinyl alcohol, polyvinyl pyrrolidone, polyvinyl methyl ether, polyethylene oxide, neutralized carbopol and xanthan gum and mixtures of said polymer.

The solid insert is preferably prepared from a cellulose derivative such as methyl cellulose, hydroxyethyl cellulose, hydroxypropyl cellulose or hydroxypropylmethyl cellulose or from other synthetic materials such as polyvinyl alcohol, polyvinyl pyrrolidone, polyethylene oxide.

Hydroxypropyl cellulose, one of the preferred polymers for the preparation of the insert is available in several polymeric forms, all of which are suitable in the preparation of these inserts. Thus, the products sold by Hercules, Inc. of Wilmington, Delaware, under the name Klucel such as Klucel HF, HWF, MF, GF, JF, LF and EF, which are intended for food or pharmaceutical use, are particularly useful. The molecular weight of these polymers useful for the purposes described herein may be at least 30,000 to about 1,000,000 or more.

Similarly, an ethylene oxide polymer having a molecular weight of up to 5,000,000 or greater, and preferably 100,000 to 5,000,000 can be employed.

Further, for example, Polyox, a polymer supplied by Union Carbide Co., may be used having a molecular weight of about 50,000 to 5,000,000 or more and preferably 3,000,000 to 4,000,000. Other specific polymers which are useful are polyvinyl pyrrolidine having a molecular weight of from about 10,000 to

2

about 1,000,000 or more, preferably up to about 350,000 and especially about 20,000 to 60,000; polyvinyl alcohol having a molecular weight of from about 30,000 to 1,000,000 or more, particularly, about 400,000 and, especially, from about 100,000 to about 200,000; hydroxypropylmethyl cellulose having a molecular weight of from about 10,000 to 1,000,000 or more, particularly, up to about 200,000 and, especially, about 80,000 to about 125,000; methyl cellulose having a molecular weight of from about 10,000 to about 1,000,000 or more, preferably up to about 200,000 and, especially, about 50,000 to 100,000; and Carbopol (carboxyvinyl polymer) of B.F. Goodrich and Co. designated as grades 934, 940 and 941.

For the purpose of this invention the type and molecular weight of the polymer are not critical. Any water soluble polymers can be used which have an average molecular weight which will afford dissolution of the polymer and, accordingly, the medicament in any desired length of time. The inserts, therefore, can be prepared to allow for retention and, accordingly, effectiveness in the eye for any desired period. The insert can be in the form of a square, rectangle, oval, circle, doughnut, semi-circle, 1/4 moon shape, and the like. Preferably, the insert is in the form of a rod, doughnut, oval or 1/4 moon. The insert can be readily prepared by mixing a polymer soluble in lacrimal fluid with an intraocular pressure reducing amount of S - (−) - 1 - (tert - butylamino) - 3 - [(4 - morpholino - 1,2,5 - thiadiazol - 3 - yl) - oxy] - 2 - propanol pamoate or its racemic modification and shaping the polymer containing the medicament as an ophthalmic insert, for example, by dissolving the medicament and the polymer in a suitable solvent and evaporating the resulting solution to afford a thin film of the polymer which can then be subdivided to prepare inserts of appropriate size. Alternatively, the insert can be prepared by intimately admixing polymer and the medicament and thereafter molding the resulting mixture under the influence of heat and pressure to form a thin film. Preferably, the inserts are prepared by molding or extrusion procedures well known in the art. The molded or extruded product can then be subdivided to afford inserts of suitable size for administration in the eye.

The insert can be of any suitable size which readily fits into the eye. For example, castings or compression molded films having a thickness of about 0.25 mm. to 1.50 mm. can be subdivided to obtain suitable inserts. Rectangular segments of the cast or compressed film having a thickness between about 0.5 and 1.5 mm. can be cut to afford shapes such as rectangular plates of 4×5—20 mm. or ovals of comparable size. Similarly, extruded rods having a diameter between about 0.5 and 1.5 mm. can be cut into suitable sections to provide the desired amount of polymer. For example, rods of 1.0 to 1.5 mm. in diameter and about 2—20 mm. long are found to be satisfactory. the inserts may also be directly formed by injection molding. It is preferred that the ophthalmic inserts containing the medicament of the present invention be formed so that they are smooth and do not have any sharp edges or corners which could cause damage to the eye. Since the terms smooth and sharp edges or corners are subjective terms, in this application these terms are used to indicate that excessive irritation of the eye will not result from the use of the insert.

The medicated ocular inserts can also contain plasticizers, buffering agents, appropriate inert fillers, and preservatives. Plasticizers suitable for this purpose must, of course, also be completely soluble in the lacrimal fluids of the eye. Examples of suitable plasticizers that might be mentioned are water, polyethylene glycol, propylene glycol, glycerine, trimethylol propane, di- and tripropylene glycol, hydroxypropyl sucrose and the like. Typically, such plasticizers can be present in the medicated ophthalmic insert in an amount ranging from 1 up to about 30% by weight. A particularly preferred plasticizer is water which is present in amounts of at least about 5%, up to about 40%. In actual practice, a water content of from about 7% to about 20% is preferred since it may be easily accomplished and adds the desired softness and pliability to the insert.

When plasticizing the solid medicinal product with water, the product is contacted with air having a relative humidity of at least 40% until said product picks up at least about 5% water and becomes softer and more pliable. In a preferred embodiment, the relative humidity of the air is from about 60% to about 99% and the contact is continued until the water is present in the product in amounts of from about 7% to about 20%.

Suitable water soluble preservatives which may be employed in the insert are alkali bisulfate, alkali thiosulfate, ascorbate, benzalkonium chloride, chlorobutanol, thimerosal, phenylmercuric acetate, phenylmercuric borate, parabens, benzyl alcohol and phenyl ethanol. These agents may be present in amounts of from 0.001 to 5% by weight of solid insert, and preferably 0.1 to 2%, and used to render the insert bacteriostatic.

Suitable water soluble buffering agents are alkali, alkali earth carbonates, phosphates, bicarbonates, citrates, borates, and the like, such as sodium or potassium phosphate, citrate, borate, acetate, bicarbonate and carbonate. These agents may be present in amounts sufficient to obtain a pH of the system of between 5.5 to 8.1 and especially 7—8; usually up to about 2% by weight of polymer. The insert may contain from about 1 mg. to 100 mg. of water soluble polymer, more particularly from 1 to 50 mg. and especially from 1 to 20 mg. The medicament is present from about 0.1 to about 25% by weight of insert.

The following examples serve to more fully illustrate this invention.

## Example 1

| S-(−)-1-(*tert*-butylamino)-3-[(4-morpholino-1,2,5-thiadiazol-3-yl)oxy]-2-propanol pamoate | 0.1 mg. |
|---|---|
| Hydroxypropylmethyl cellulose q.s. | 12 mg. |

Ophthalmic inserts are manufactured from a solvent cast film prepared by making a viscous solution of the powder blend using a methanol/water solvent system (10 ml. methanol is added to 2,5 g. of powder blend, to which 11 ml. of water (in three divided portions) is added. The solution is placed on a Teflon® (polytetrafluoroethylene) plate and allowed to dry at ambient conditions. After drying, the film is placed in an 88% relative humidity cabinet until it is pliable. Appropriately sized inserts are then cut from the film.

## Example 2

| S-(−)-1-(*tert*-butylamino)-3-[(4-morpholino-1,2,5-thiadiazol-3-yl)oxy]-2-propanol pamoate | 0.1 mg. |
|---|---|
| Hydroxypropyl cellulose q.s. a.d. | 12 mg. |

Ophthalmic inserts are manufactured from compression molded films which are prepared on a Carver Press by subjecting the powder mixture of the above ingredients to a compressional force of 12,000 lbs. (gauge) at 350°F for one minute. The film is cooled under pressure by having cold water circulate in the platen. Ophthalmic inserts are then individually cut from the film with a punch. Each insert is placed into a vial, which is then placed in a humidity cabinet (88% relative humidity at 30°C) for two to four days. After removal from the humidity cabinet, the vials are stoppered and then capped. The vials containing the hydrated insert are then sterilized by means known in the art such as irradiation with high energy electron beams, gamma radiation with a suitable radiation source such as $Co^{60}$.

When using inserts to administer the medication, one insert per day per eye is suitable. However, as with all medication, medical monitoring of symptoms must be employed to enable one to arrive at the optimum dosage for the patient.

Example 3

S - (−) - 1 - (*tert* - butylamino) - 3 - [(4 - morpholino - 1,2,5 - thiadiazol - 3 - yl) - oxy] - 2 - propanol pamoate (2:1 salt) is prepared in the following manner:

11 g. of S - (−) - 1 - (*tert* - butylamino) - 3 - [(4 - morpholino - 1,2,5 - thiadiazol - 3 - yl) - oxy] - 2 - propanol maleate (1:1 salt) is dissolved in 100 ml of distilled water, and 5 g. of disodium pamoate are dissolved in 100 ml of distilled water. It is entirely suitable to institute sterile filtration of the S - (−) - 1 - (*tert* - butylamino) - 3 - [(4 - morpholino - 1,2,5 - thiadiazol - 3 - yl) - oxy] - 2 - propanol maleate solution and disodium pamoate solution prior to combination in sterile vessels. Subsequently these solutions are aseptical and a sterile product results which can be aseptically formulated for suspensions, and the like. Inserts, of course, are preferably sterilized by irradiation techniques. The two solutions are combined and stirred for a sufficient time for precipitation to become complete, usually 15 minutes will suffice. The suspension of precipitate and other liquid is then filtered through a suitable filter such as a sintered glass funnel with vacuum applied to hasten the process. The filter cake is washed until any residual timolol maleate is removed. The product is then dried and the drying procedure must be carried out at a temperature less than 30°C to preclude formation of unsuitable material which unsuitable material is oil-like and gummy.

Example 4

The S - (−) - 1 - (*tert* - butylamino) - 3 - [(4 - morpholino - 1,2,5 - thiadiazol - 3 - yl) - oxy] - 2 - propanol pamoate from the above example is admixed with hydroxypropyl cellulose in the following manner:

To prepare one preferred embodiment of the inserts of this invention, 1.75 g. of the above mixture from Example 1 is compression molded. A 2"×2"×0.6 mm film using 300°F for 1.5 minutes at 10 to 12,000 pounds (gauge) and then cooling for 2.5 minutes at 10 to 12,000 pounds (gauge). Cut inserts 12.5 mm long×2.5 mm wide×0.6 mm thick. Place inserts in a relative humidity atmosphere of 88% (R.T.). Place hydrate inserts in 1 cc glass vials and stopper with 830 pink butyl or other suitable closure.

These inserts were compared in rabbits to inserts containing S - (−) - 1 - (*tert* - butylamino) - 3 - [(4 - morpholino - 1,2,5 - thiadiazol - 3 - yl) - oxy] - 2 - propanol maleate in Klucel GF and to timolol maleate drops. It was concluded that in the α-chymotrypsin induced glaucoma model, S - (−) - 1 - (*tert* - butylamino) - 3 - [(4 - morpholino - 1,2,5 - thiadiazol - 3 - yl) - oxy] - 2 - propanol insert formulations induced a greater and more prolonged intraocular pressure (IOP) drop than timolol solution. S - (−) - 1 - (*tert* - butylamino) - 3 - [(4 - morpholino - 1,2,5 - thiadiazol - 3 - yl) - oxy] - 2 - propanol pamoate inserts were clearly more active than S - (−) - 1 - (*tert* - butylamino) - 3 - [(4 - morpholino - 1,2,5 -

# 0 034 542

thiadiazol - 3 - yl) - oxy] - 2 - propanol maleate inserts with respect to both amplitude and duration of the ocular hypotensive effect.

**Claims for the Contracting States: BE CH DE FR GB IT LI LU NL SE**

1. An ophthalmic composition for lowering intraocular pressure comprising in a solid ophthalmic insert soluble in lacrimal fluids an intraocular pressure reducing amount of S - (−) - 1 - (tert - butylamino) - 3 - [(4 - morpholino - 1,2,5 - thiadiazol - 3 - yl) - oxy] - 2 - propanol pamoate or its racemic modification.

2. A composition according to claim 1 where there is included in the composition a preservative so that said composition is bacteriostatic.

3. A composition according to claims 1 or 2 where the concentration of S - (−) - 1 - (tert - butylamino) - 3 - [(4 - morpholino - 1,2,5 - thiadiazol - 3 - yl) - oxy] - 2 - propanol pamoate or its racemic modification is from 0.01 to 50% by weight of said composition.

**Claims for the Contracting State: AT**

1. A process for preparation of a solid ophthalmic insert for lowering intraocular pressure characterised by mixing a polymer soluble in lacrimal fluid with an intraocular pressure reducing amount of S - (−) - 1 - (tert - butylamino) - 3 - [(4 - morpholino - 1,2,5 - thiadiazol - 3 - yl) - oxy] - 2 - propanol pamoate or its racemic modification. and shaping the polymer containing the medicament as an ophthalmic insert.

2. A process according to claim 1 wherein a preservative is incorporated during mixing.

3. A process according to claim 1 or 2 where the concentration of S - (−) - 1 - (tert - butylamino) - 3 - [(4 - morpholino - 1,2,5 - thiadiazol - 3 - yl) - oxy] - 2 - propanol pamoate or its racemic modification is from 0.01 to 50% by weight of said composition.

**Patentansprüche für die Vertragsstaats: BE CH DE FR GB IT LI LU NL SE**

1. Ophthalmische Zusammensetzung zur Verringerung des intraokularen Drucks, enthaltend in einer festen, in Tränenflüssigkeit löslichen, ophthalmischen Einlage, eine den intraokularen Druck verringernde Menge von S - (−) - 1 - (tert-Butylamino) - 3 - [(4 - morpholino - 1,2,5 - thiadiazol - 3 - yl) - oxy] - 2 - propanol - pamoat oder seine racemische Modifikation.

2. Zusammensetzung nach Anspruch 1, wobei in die Zusammensetzung ein Konservierungsmittel einbezogen ist, so daß die Zusammensetzung bakteriostatisch ist.

3. Zusammensetzung nach Anspruch 1 oder 2, worin die Konzentration an S - (−) - 1 - (tert - Butylamino) - 3 - [(4 - morpholino - 1,2,5 - thiadiazol - 3 - yl) - oxy] - 2 - propanol - pamoat oder seiner racemischen Modifikation 0,01 bis 50 Gew.-% der Zusammensetzung beträgt.

**Patentansprüche für den Vertragsstaat: AT**

1. Verfahren zur Herstellung einer festen, den intraokularen Druck senkenden, ophthalmischen Einlage, dadurch gekennzeichnet, daß man ein Polymeres, das in Tränenflüssigkeit löslich ist, mit einer den intraokularen Druck verringernden Menge von S - (−) - 1 - (tert - Butylamino) - 3 - [(4 - morpholino - 1,2,5 - thiadiazol - 3 - yl) - oxy] - 2 - propanol - pamoat oder seiner racemischen Modifikation vermischt und das das Arzneimittel enthaltende Polymere zu einer ophthalmischen Einlage formt.

2. Verfahren nach Anspruch 1, bei dem ein Konservierungsmittel während des Vermischens eingearbeitet wird.

3. Verfahren nach Anspruch 1 oder 2, bei dem die Konzentration an S - (−) - 1 - (tert - Butylamino) - 3 - [(4 - morpholino - 1,2,5 - thiadiazol - 3 - yl) - oxy] - 2 - propanol - pamoat oder seiner racemischen Modifikation 0,01 bis 50 Gew.-% der Zusammensetzung beträgt.

**Revendications pour les Etats Contractants: BE CH DE FR GB IT LI LU NL SE**

1. Une composition ophtalmique pour abaisser la pression intra-oculaire comprenant, dans un insert ophtalmique solide soluble dans les liquides lacrymaux, une quantité abiassant la pression intra-oculaire de pamoate de S - (−) - 1 - (tert - butylamino) - 3 - [(4 - morpholino - 1,2,5 - thiadiazol - 3 - yl)oxy] - 2 - propanol ou de sa modification racémique.

2. Une composition selon la revendication 1, dans laquelle un conservateur est incorporé à la composition pour que ladite composition soit bactériostatique.

3. Une composition selon les revendications 1 ou 2, dans laquelle la concentration du pamoate de S - (−) - 1 - (tert - butylamino) - 3 - [(4 - morpholino - 1,2,5 - thiadiazol - 3 - yl)oxy] - 2 - propanol ou de sa modification racémique est de 0,01 à 50% du poids de ladite composition.

**Revendications pour l'Etat Contractant: AT**

1. Un procédé pour la préparation d'un insert ophtalmique solide pour abaisser la pression

**0 034 542**

intraoculaire, caractérisé par le mélange d'un polymère soluble dans le liquide lacrymal avec une quantité abaissant la pression intra-oculaire de pamoate de S - (−) - 1 - (tert - butylamino) - 3 - [(4 - morpholino - 1,2,5 - thiadiazol - 3 - yl)oxy] - 2 - propanol ou de sa modification racémique, et le façonnage du polymère contenant le médicament sous forme d'un insert ophtalmique.

2. Un procédé selon la revendication 1, dans lequel un conservateur est incorporé au cours du mélange.

3. Un procédé selon la revendication 1 ou 2, dans lequel la concentration du pamoate de S - (−) - 1 - (tert - butylamino) - 3 - [(4 - morpholino - 1,2,5 - thiadiazol - 3 - yl)oxy] - 2 - propanol ou de sa modification racémique est de 0,01 à 50% du poids de ladite composition.